# EUROPEAN PATENT APPLICATION

(11) **EP 4 279 595 A1**
(43) Date of publication of application: **22.11.2023**
(21) Application number: 22736513.7
(22) Date of filing: 05.01.2022
(51) Int. Cl.: C12N 15/53, C12N 9/02, C12N 15/72, C12N 1/21, C12P 7/62, C12P 7/02, C12P 17/18, C12R 1/19

(54) **POLYNUCLEOTIDE AND EXPRESSION CASSETTE COMPRISING CODING SEQUENCE OF TOLUENE DIOXYGENASE, AND USE THEREOF**

(30) Priority: 06.01.2021 CN 202110015598
(71) Applicant: Shanghai Shengping Medical Equipment Co., Ltd., Shanghai 200030 (CN); Shanghai Vastpro Technology Development Co., Ltd., Shanghai 201210 (CN); Zhejiang Boxiao Biopharmaceutical Co., Ltd., Hangzhou, Zhejiang 311404 (CN)
(72) Inventor: YANG, Yong, Shanghai 201210 (CN); HUANG, Ping, Shanghai 200030 (CN); JIANG, Biao, Shanghai 201210 (CN); ZHU, Wenfeng, Shanghai 201210 (CN)
(74) Representative: Müller, Christian Stefan Gerd
(86) International application number: PCT/CN2022/070188
(87) International publication number: WO 2022/148351

(57) **Abstract**

The present disclosure relates to the field of bioengineering and pharmaceutical and chemical production. In particular, provided is a polynucleotide comprising a coding sequence of toluene dioxygenase. Also provided are an expression cassette, a vector and a host cell comprising the polynucleotide, as well as use thereof in the preparation of cis-cyclohexadiene o-diol compounds.

## Description

The present application claims priority of Chinese Patent Application No. 202110015598.1, filed on January 6, 2021, with the title of "Polynucleotides and Expression Cassettes Comprising Coding Sequences of Toluene Dioxygenase, and Uses Thereof', of which the content is incorporated herein in its entirety.

### Technical Field

The present disclosure relates to the field of bioengineering and pharmaceutical and chemical production. In particular, provided is a polynucleotide comprising a coding sequence of toluene dioxygenase. Also provided are also an expression cassette, a vector and a host cell comprising the polynucleotide, as well as use thereof in the preparation of cis-cyclohexadiene o-diol compounds.

### Background

R. E. Kallio *et al.* found a class of aromatic ring-dihydroxylating oxygenases in aromatic hydrocarbon-degrading bacteria in the late 1960s (Gibson, D. T. et al., Biochemistry, 1968, 7, 2653). These enzymes use substrates such as benzene, toluene, naphthalene and biphenyl to achieve prokaryotic metabolism of aromatic rings through aromatic nucleus activation, ring fission and citric acid cycle intermediates. This research is important for understanding and utilizing the natural carbon cycle processes and also have applications in synthesis of unique chiral chemical intermediates. Toluene dioxygenase belongs to a class of biocatalysts which have been widely studied and is beneficial for performing important functional group transformations such as 1) aromatic cis-dihydroxylation, 2) olefin cis-dihydroxylation, 3) sulfide oxidation, 4) benzyl hydroxylation, and 5) hydrocarbon desaturation and N-dealkylation. The aromatic ring is exclusively converted to the unique chiral metabolite cis-cyclohexa-3,5-diene-1,2-diol or cis-dihydrodiol intermediates with the action of toluene oxygenase, and such enzymatic reaction provides a new pathway for the synthetic chemistry of complex molecules with chiral cis-dihydrodiols as precursors. Tomas Hudlicky *et al.* reported in 2018 the conversion of toluene or its analogues to cis-cyclohexadiene o-diol intermediates by a double hydroxylation reaction via enzymatic whole-cell fermentation using a toluene dioxygenase strain of *E. coli* JM109 (pDTG601) (Baidilov, D. et al., Angew.Chem. Int. Ed. 2018, 57, 10994-10998). They concluded from literature analysis and suggested that the natural product Tetrodotoxin could be synthesized from this intermediate by a 21-step reaction as reported by Fukuyama *et al.*

The DNA of the toluene dioxygenase system beared by the expression plasmid pDTG601 used by Tomas Hudlicky *et al.* was directly derived from the original sequence on *Pseudomonas putida* F1 genome, which contains not only the coding sequences of the four genes *todC1*/*todC2*/*todB*/*todA*, but also the spacer sequences among the coding sequences and the combination of the four coding sequences. In general, direct use of the DNA sequence from a different species for expression in *E. coli* may result in unsatisfactory protein expression due to codon usage bias, species differences in promoter and ribosome recognition sites, thereby affecting the catalytic effect of the enzyme.

### Summary

In an aspect, provided is a polynucleotide, comprising coding sequences of toluene dioxygenase TodC1, TodC2, TodB and TodA.

In an embodiment, the polynucleotide comprises, from 5'-end to 3'-end: (1) a first nucleotide sequence, comprising the coding sequence of TodC1, wherein the TodC1 has an amino acid sequence of SEQ ID NO: 4; (2) a second nucleotide sequence, comprising the coding sequence of TodC2, wherein the TodC2 has an amino acid sequence of SEQ ID NO: 5; (3) a third nucleotide sequence, comprising the coding sequence of TodB, wherein the TodB has an amino acid sequence of SEQ ID NO: 6; and (4) a fourth nucleotide sequence, comprising the coding sequence of TodA, wherein the TodA has an amino acid sequence of SEQ ID NO: 7; wherein the first, second, third and fourth nucleotide sequences each independently use *E. coli* preferred codons, and the stop codon TAA for TodB and the start codon ATG for TodA form a nested sequence TAATG.

In a specific embodiment, the polynucleotide comprises a nucleotide sequence of SEQ ID NO: 1 or SEQ ID NO: 2.

In another aspect, also provided are an expression cassette or vector, comprising the polynucleotide according to the present disclosure and a host cell comprising the polynucleotide, expression cassette or vector according to the present disclosure.

In another aspect, provided is a process for preparing a toluene dioxygenase, comprising culturing the host cell according to the present disclosure under conditions suitable for the expression of the expression cassette or vector.

In another aspect, provided is a process for preparing a compound of formula (2), 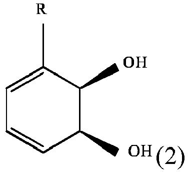 the process comprising,
(1) culturing the host cell according to the present disclosure under conditions suitable for the expression of the expression cassette or vector;
(2) adding a compound of formula (1) as substrate to the culture medium containing the host cell of step (1) for reaction to give a fermentation broth containing the compound of formula (2), 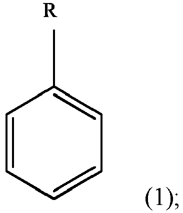 and
(3) recovering the compound of formula (2) from the fermentation broth of step (2);

wherein R is selected from the group consisting of halogen, -C₁₋₆ alkyl, -C₂₋₆ alkenyl, -C₁₋₆ alkyl-CN, -C₁₋₆ alkyl-O-C(=O)-C₁₋₆ alkyl, -C₃₋₈ cycloalkyl, -C₃₋₈ cycloalkenyl, and -C₆₋₁₀ aryl, wherein the C₁₋₆ alkyl, -C₂₋₆ alkenyl, -C₁₋₆ alkyl-CN, -C₁₋₆ alkyl-O-C(=O)-C₁₋₆ alkyl, -C₃₋₈ cycloalkyl, -C₃₋₈ cycloalkenyl, and -C₆₋₁₀ aryl are optionally substituted with one or more halogen;
preferably, R is selected from the group consisting of halogen, methyl, -CH=CH₂, -CH=CHBr, -CH₂CH₂Br, -CH₂CH₂CN, -CH₂CH₂N₃, -CH₂CH₂-O-C(=O)-CH₃, -CH₂-O-C(=O)-CH₃, cyclohexyl, cyclohexenyl, and phenyl.

Accordingly, in another aspect, provided is also use of the polynucleotide, the expression cassette or vector, or the host cell according to the present disclosure for preparing a compound of formula (2).

### Brief Description of the Drawings

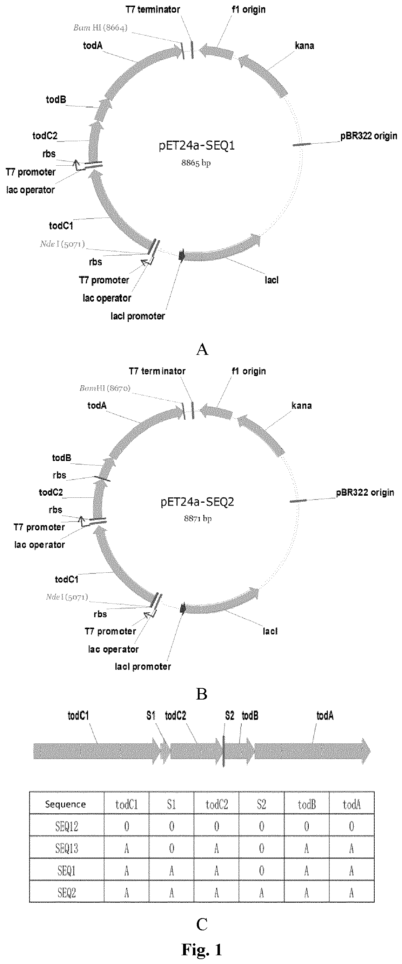

Figure 1: A and B are plasmid profiles of pET-24a-SEQ1 and pET-24a-SEQ2, respectively. The fragment contained between the NdeI and BamHI enzymatic sites is the polynucleotide according to the present disclosure. C shows the sequence annotation of SEQ1, SEQ2, SEQ12 and SEQ13; where S1 and S2 represent the first spacer sequence and the second spacer sequence, respectively; O represents the original sequence from the genome of *Pseudomonas putida* F1 strain, and A represents the artificially designed sequence.

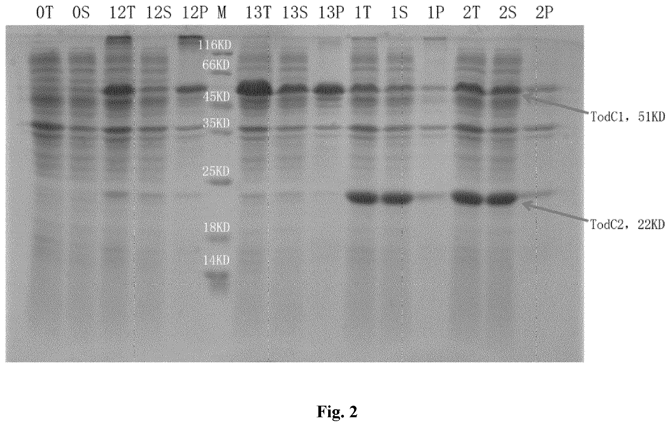

Figure 2: SDS-PAGE protein gel electrophoresis analysis shows soluble expression of toluene dioxygenase in the recombinant strains, where the Coomassie brilliant blue staining shows protein bands representing TodC1 and TodC2; 0, 12, 13, 1 and 2 represent recombinant strains BL21(DE3)(pET-24a), BL21(DE3)(pET-24a-SEQ12), BL21(DE3)(pET-24a-SEQ13), BL21(DE3)(pET-24a-SEQ1) and BL21(DE3)(pET-24a-SEQ2), respectively. T, S and P represent whole cells, supernatants and precipitates, respectively.

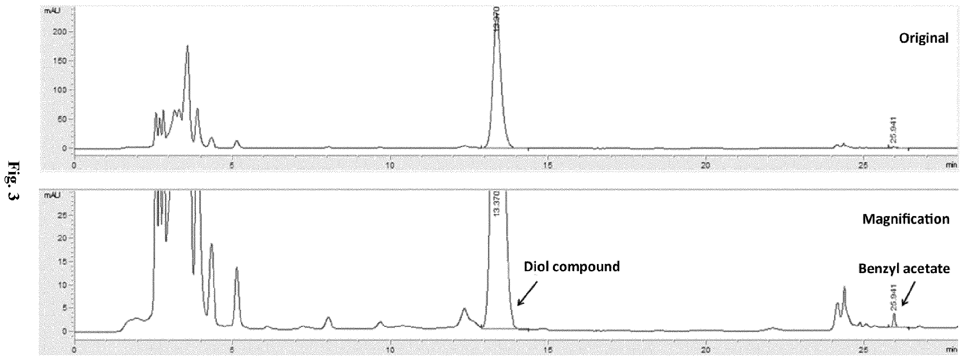

Fig. 3: HPLC chromatogram during biotransformation using strain BL21(DE3)(pET-24a-SEQ1) with benzyl acetate as substrate, with the diol compound product cis-(1S,2R)-3-acetoxymethyl-3,5-cyclohexadiene-1,2-diol (CAS:131043-51-1) at 13 min and the substrate benzyl acetate at 25 min.

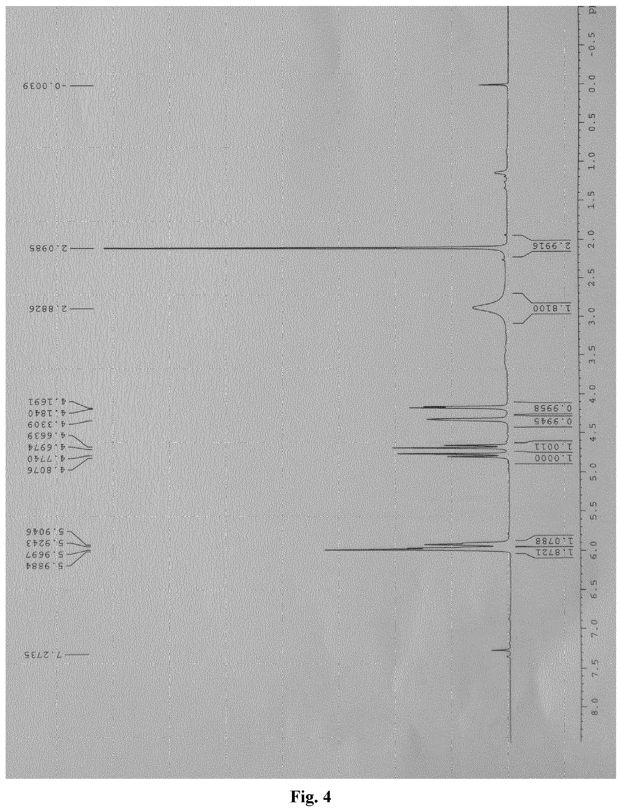

Figure 4: NMR pattern of the product cis-(1S,2R)-3-acetoxymethyl-3,5-cyclohexadiene-1,2-diol (CAS: 131043-51-1) obtained through biotransformation with strain BL21(DE3)(pET-24a-SEQ1) using benzyl acetate as substrate. The solvent is deuterated chloroform.

### Detailed Description

The technical contents of the present disclosure are described with specific embodiments, and other advantages of the present disclosure can be readily appreciated by those skilled in the art according to the disclosure of the present specification. The present disclosure may also be implemented or applied with other specific embodiments. Those skilled in the art can make various modifications and changes without departing from the spirit of the present disclosure. Unless explicitly stated, the reagents and apparatus used in the following examples are conventional in the field and are commercially available. The procedures as used are also conventional, and those skilled in the art can clearly know how to conduct the experiments as described and obtain the corresponding results according to the specification.

### General terms and definitions

Unless otherwise stated, scientific and technical terms used herein have the meanings commonly understood by those skilled in the art. Furthermore, the terms of protein and nucleic acid chemistry, molecular biology, cell and tissue culture, and microbiology used herein are those widely used in the fields. Moreover, definitions and explanations of relevant terms are provided below for a better understanding of the present disclosure.

As used herein, unless the context indicates otherwise, the singular form of the expression "a", "an" or "the" encompasses the plural designation.

The ranges herein encompass the endpoints and each point value therein as well as the subranges formed by these values. For example, C₁₋₆ encompasses C₁, C₂, C₃, C₄, C₅, C₆, C₁₋₅, C₂₋₆, C₃₋₅, C₂₋₄ or the like. As another example, C₃₋₈ encompasses C₃, C₄, C₅, C₆, C₇, C₈, C₃₋₇, C₄₋₈, C₅₋₇ or the like.

As used herein, the term "one or more" or "at least one" encompasses one, two, three, four, five, six, seven, eight, nine or more.

As used herein, a "nucleotide" comprises a deoxyribonucleotide and a ribonucleotide and a derivative thereof. A "nucleotide" is usually designated by the single letter representing the base therein: "A(a)" refers to deoxy-adenylate or adenylate, "C(c)" refers to deoxy-cytidylate or cytidylate, "G(g)" refers to deoxy-guanylate or guanylate, "U(u)" refers to uridylate, "T(t) " refers to deoxy-thymidylate.

As used herein, the term "polynucleotide" refers to a polymer of deoxyribonucleotides (DNA) or a polymer of ribonucleotides (RNA). The terms "polynucleotide sequence", "nucleic acid sequence" and "nucleotide sequence" are used interchangeably to denote the sequence of nucleotides in a polynucleotide. A polynucleotide can be single-stranded or double-stranded. A polynucleotide can also contain a synthetic, non-natural, or modified nucleotide. A polynucleotide can contain one or more nucleotide sequences (e.g., 1, 2, 3, 4, 5, 6, 7, 8 sequences). A nucleotide sequence may comprise but be not limited to, for example, a coding sequence encoding a protein or polypeptide, a regulatory sequence regulating the expression of a coding sequence, and a restriction endonuclease cleavage site.

As used herein, a "codon" refers to a sequence of three consecutive nucleotides (also known as triplet codon) which encodes a specific amino acid in a polynucleotide. The use of codons may be biased in different species, i.e., synonymous codons (codons encoding the same amino acid) are used in different species with different frequencies. It is generally believed that protein coding sequences using preferred codons for a species can be translated with high efficiency and accuracy in the expression system of that species. The term "E. *coli* preferred codons" refers to codons that are used with higher frequencies in *E. coli.* The coding sequence contained in the polynucleotide according to the present disclosure uses *E. coli* preferred codons.

As used herein, the term "polypeptide" refers to a polymer containing more than two amino acids covalently linked by peptide bonds. A "protein" may contain one or more polypeptides, wherein the polypeptides interact with each other in a covalent or non-covalent manner. Unless otherwise specified, the terms "polypeptide" and "protein" may be used interchangeably.

As used herein, "isolated" means that a substance (e.g., a polynucleotide or polypeptide) is separated from the source or environment in which it exists, i.e., it is substantially free of other ingredients.

As used herein, the term "expression cassette" refers to a polynucleotide which is capable of directing the expression of a target coding sequence and comprises a target coding sequence operably linked to a regulatory sequence. The expression cassette according to the present disclosure is used to express a protein or RNA in an *E. coli* expression system. Regulatory sequences which can be used in the expression cassette according to the present disclosure may include, for example, a promoter, a transcription factor binding site (e.g., a ribosome binding site), an enhancer, and a transcription termination signal (e.g., a transcription terminator).

As used herein, "operably linked" means that nucleotide sequences are linked or adjacent to each other, such that the function of a nucleotide sequence is affected by another. For example, a promoter which is operably linked to a target coding sequence directs the transcription of the target coding sequence, and a ribosome binding site which is operably linked to a target coding sequence directs the translation of the target coding sequence.

As used herein, a "coding sequence" refers to a nucleotide sequence encoding a target protein (e.g., TodC1, TodC2, TodB and TodA) or polypeptide.

As used herein, the term "expression" refers to the process for producing a protein from a polynucleotide (e.g., a target coding sequence). In some embodiments, the expression vector according to the present disclosure is "inducible expression". "Inducible expression" means that the expression of a gene needs to be achieved under specific conditions (e.g., in the presence of an inducer). For example, in some embodiments according to the present disclosure, the expression of toluene dioxygenase TodC 1 C2BA is induced by addition of an inducer (e.g., IPTG) to the culture medium of a recombinant strain.

As used herein, the term "promoter" refers to a nucleotide sequence comprising a sequence recognized by an RNA polymerase, which controls the expression of a coding sequence by containing a recognition sequence for an RNA polymerase and other factors required for proper transcription. A "promoter regulatory sequence" may comprise a proximal and more distal upstream and/or downstream element. A promoter regulatory sequence affects the transcription, RNA processing or stability, or translation of a coding sequence operably linked thereto. The meaning of the term "promoter" encompasses "promoter regulatory sequence".

As used herein, an "enhancer" is a nucleotide sequence which may enhance the transcription of a target coding sequence. An enhancer may be homologous or heterologous to a promoter. It can be located upstream or downstream of the promoter sequence.

As used herein, "transformation" refers to the introduction of a polynucleotide or vector into a host cell such that the polynucleotide or vector can be expressed from the host cell. Transformation may be transient or stable. A host cell transformed with the polynucleotide, expression cassette or vector according to the present disclosure may also be referred to as a "recombinant cell". In some embodiments, the host cell is a bacterial strain such as *E. coli,* and such a recombinant cell may also be referred to as a "recombinant strain".

As used herein, the term "host cell" refers to a cell which is used to receive, maintain, replicate, or express a polynucleotide or vector. As used herein, a host cell is preferably a cell which can express the target coding sequence.

The term "toluene dioxygenase" or "TodC1C2BA" refers to the combined name of *Pseudomonas putida* F1 strain proteins TodC1, TodC2, TodB and TodA. The nucleotide sequences of *todC1*, *todC2*, *todB* and *todA* genes of *Pseudomonas putida* F1 are shown in nucleotides at positions 620-4210 of GenBank accession number: J04996.1 (SEQ ID NO: 12), which comprises spacer sequences. "TodC1" is encoded by *todC1* (also known as *bnzA*) gene, which is also known as Benzene 1,2-dioxygenase subunit alpha, having an amino acid sequence as shown in Uniprot ID: A5W4F2 (SEQ ID NO: 4). "TodC2" is encoded by *todC2* (also known as *bnzB*) gene, which is also known as Benzene 1,2-dioxygenase subunit beta, having an amino acid sequence as shown in Uniprot ID: A5W4F1 (SEQ ID NO: 5). "TodB" is encoded by *todB* gene, which is also known as Toluene 1,2-dioxygenase system ferredoxin subunit, having an amino acid sequence as shown in Uniprot ID: A5W4F0 (SEQ ID NO: 6). "TodA" is encoded by *todA* gene, which is also known as Toluene 1,2-dioxygenase system ferredoxin--NAD(+) reductase component, having an amino acid sequence as shown in Uniprot ID: A5W4E9 (SEQ ID NO: 7).

TodC1C2BA co-catalyzes a reaction from toluene or toluene analog (Formula (1)) to cis-cyclohexadiene o-diol compounds (Formula (2)) as shown below: 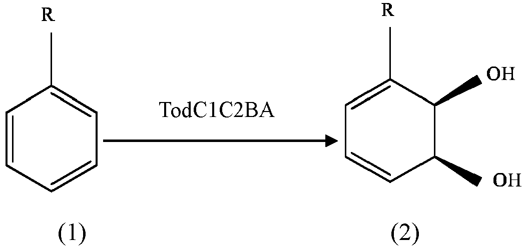 wherein R may be halogen, methyl, -CH=CH₂, -CH=CHBr, -CH₂CH₂Br, -CH₂CH₂CN, -CH₂CH₂N₃, -CH₂CH₂-O-C(=O)-CH₃, -CH₂-O-C(=O)-CH₃, cyclohexyl, cyclohexenyl, or phenyl. The products obtained from different substrates catalyzed by toluene dioxygenase can be used as intermediates for the synthesis of various compounds, and relevant description can be found in e.g., Endoma, M. A., et al., Organic Process Research & Development, 2002, 6(4): 525-532.

As used herein, "toluene or toluene analog" has the structure shown in Formula (1) above.

As used herein, the term "cis-cyclohexadiene o-diol compound" has the structure shown in Formula (2) above.

As used herein, the term "substitution" indicates that one or more specific hydrogen atoms are replaced by selected group(s), provided that the normal atomic valence of the specific atom is not exceeded and that the substitution forms a stable compound. Combination of substituents and/or variables are permitted only if such combination forms a stable compound. Examples of substituents of a optionally substituted group herein may be halogen. The alkyl, alkenyl, cycloalkyl, cycloalkenyl, aryl as described herein may be optionally substituted.

As used herein, the term "halogen" refers to fluorine (F), chlorine (Cl), bromine (Br), and iodine (I).

As used herein, "i-j" or a similar expression refers to a range covering the endpoints, each integer therein, and subranges formed by the integers therein. For example, C₁₋₆ encompasses C₁, C₂, C₃, C₄, C₅ and C₆ as well as C₂₋C₅, C₃₋C₄ or the like.

As used herein, the term "alkyl", either alone or in combination with other terms, refers to a branched or straight-chain saturated aliphatic hydrocarbon group having a specific number of carbon atoms. Unless otherwise indicated, "alkyl" refers to C₁₋₆ alkyl. For example, "C₁₋₆ alkyl" includes but is not limited to methyl, ethyl, n-propyl, isopropyl, n-butyl, tert-butyl, isobutyl, pentyl, hexyl, or the like.

As used herein, the term "alkenyl", either alone or in combination with other terms, refers to a branched or straight-chain unsaturated aliphatic hydrocarbon group having a specific number of carbon atoms with a carbon-carbon double bond. Unless otherwise indicated, "alkenyl" refers to C₂₋₆ alkenyl.

As used herein, the term "cycloalkyl", either alone or in combination with other terms, refers to a saturated aliphatic cyclic hydrocarbon group having a specific number of carbon atoms. Unless otherwise indicated, "cycloalkyl" refers to C₃₋₈ cycloalkyl. Examples comprise but are not limited to cyclopropyl, cyclobutyl, cyclopentyl, cyclohexyl or the like.

As used herein, the term "cycloalkenyl", either alone or in combination with other terms, refers to an unsaturated aliphatic cyclic hydrocarbon group having a specific number of carbon atoms with a carbon-carbon double bond. Unless otherwise indicated, "cycloalkenyl" refers to C₃₋₈ cycloalkenyl. Examples comprise but are not limited to cyclopropenyl, cyclobutenyl, cyclopentenyl, cyclohexenyl or the like.

As used herein, the term "aryl", either alone or in combination with other terms, refers to an aromatic hydrocarbon group containing a 5- or 6-membered aromatic carbon ring. Unless otherwise indicated, "aryl" refers to C₆₋₁₀ aryl. Examples comprise but are not limited to phenyl, naphthyl or the like.

The DNA recombination techniques used herein are well known in the field (see, e.g., Sambrook, J. et al., Molecular Cloning: A Laboratory Manual, 2nd ed., Cold Spring Harbor Laboratory Press, Cold Spring Harbor, N.Y, 1989).

### Polynucleotides

In an aspect, provided is a polynucleotide, comprising coding sequences of toluene dioxygenase of *Pseudomonas putida* F1 strain TodC1, TodC2, TodB and TodA. Preferably, the polynucleotide according to the present disclosure is isolated.

In an embodiment, the polynucleotide comprises, from 5'-end to 3'-end:
(1) a first nucleotide sequence, comprising the coding sequence of TodC1, wherein the TodC1 has an amino acid sequence of SEQ ID NO: 4;
(2) a second nucleotide sequence, comprising the coding sequence of TodC2, wherein the TodC2 has an amino acid sequence of SEQ ID NO: 5 ;
(3) a third nucleotide sequence, comprising the coding sequence of TodB, wherein the TodB has an amino acid sequence of SEQ ID NO: 6; and
(4) a fourth nucleotide sequence, comprising the coding sequence of TodA, wherein the TodA has an amino acid sequence of SEQ ID NO: 7;
wherein the first, second, third and fourth nucleotide sequences each independently use *E*. *coli* preferred codons, and the stop codon TAA for TodB and the start codon ATG for TodA form a nested sequence TAATG.

In a preferable embodiment, the first nucleotide sequence comprises nucleotides at positions 1-1350 of SEQ ID NO: 1 (see also, nucleotides at positions 1-1350 of SEQ ID NO: 2). In another preferable embodiment, the second nucleotide sequence comprises nucleotides at positions 1464-2024 of SEQ ID NO: 1 (see also, nucleotides at positions 1464-2024 of SEQ ID NO: 2). In another preferable embodiment, the third nucleotide sequence comprises nucleotides at positions 2036-2356 of SEQ ID NO: 1 (see also, nucleotides at positions 2042-2362 of SEQ ID NO: 2). In another embodiment, the fourth nucleotide sequence comprises nucleotides at positions 2359-3588 of SEQ ID NO: 1 (see also, nucleotides at positions 2365-3594 of SEQ ID NO: 2).

In some embodiments, the polynucleotide further comprises a first spacer sequence located between the first nucleotide sequence and the second nucleotide sequence, and/or a second spacer sequence located between the second nucleotide sequence and the third nucleotide sequence.

In an embodiment, the first spacer sequence comprises, from 5'-end to 3'-end, a T7 promoter, a Lac operator (LacO) and a prokaryotic ribosome binding site (rbs). In an embodiment, the T7 promoter comprises a nucleotide sequence of SEQ ID NO: 8. In an embodiment, the prokaryotic ribosome binding site is rich in AG, e.g., AAGGAG. In a specific embodiment, the prokaryotic ribosome binding site comprises a nucleotide sequence of SEQ ID NO: 9. In an embodiment, the Lac operator comprises a nucleotide sequence of SEQ ID NO: 10.

In an embodiment, the second spacer sequence comprises GTGATGTC (nucleotides at positions 2028-2035 of SEQ ID NO: 1). In a preferable embodiment, the second spacer sequence comprises an rbs sequence rich in AG, e.g., AAGGAG. In a more preferable embodiment, the second spacer sequence comprises a nucleotide sequence of SEQ ID NO: 9.

Preferably, the first spacer sequence comprises a nucleotide sequence of SEQ ID NO: 3, and/or the second spacer sequence comprises GTGATGTC (nucleotides at positions 2028-2035 of SEQ ID NO: 1) or GAAGGAGATATACC (nucleotides at positions 2028-2041of SEQ ID NO: 2, corresponding to SEQ ID NO: 9).

In a specific embodiment, the polynucleotide comprises a nucleotide sequence of SEQ ID NO: 1 or SEQ ID NO: 2.

In an embodiment, the first, second, third and fourth nucleotide sequence further each independently comprises a coding sequence of a polypeptide tag. The coding sequence of the polypeptide tag is operably linked to the coding sequence of the target polypeptide (e.g., coding sequence of TodC1, TodC2, TodB or TodA), such that the polypeptide tag and the target polypeptide are expressed as a fusion polypeptide from the same open reading frame. Examples of polypeptide tags comprise but are not limited to, glutathione S-transferase (GST), polyhistidine tag (i.e., His tag, e.g., His₅, His₆, His₈, etc.), maltose binding protein (MBP), B1 domain of streptococcal G protein (GB1), thioredoxin (TRX), and small ubiquitin-related modified protein (SUMO).

### Expression Cassettes and Vectors

In another aspect, provided is a vector, comprising the polynucleotide according to the present disclosure.

The vector may be a cloning vector or an expression vector. In a preferable embodiment, the vector is an expression vector.

In a preferable embodiment, the vector comprises the polynucleotide according to the present disclosure in the form of an expression cassette, and such a vector can be an expression vector. Accordingly, in another aspect, provided is an expression cassette, comprising the polynucleotide according to the present disclosure and a regulatory sequence. The regulatory sequence is operably linked to the polynucleotide according to the present disclosure and directs the expression of the protein of interest (e.g., toluene dioxygenase TodC1, TodC2, TodB and TodA). Regulatory sequences suitable for the expression cassette may include but be not limited to a promoter, a transcriptional enhancer, a translational enhancer, a prokaryotic ribosome binding site, a regulatory element of Lac operon, a transcription terminator and a combination thereof. In a preferable embodiment, the regulatory sequence is a promoter, a prokaryotic ribosome binding site, a transcription terminator or a combination thereof. In a more preferable embodiment, the regulatory sequence is a promoter, a regulatory element of Lac operon, a prokaryotic ribosome binding site, a transcription terminator or a combination thereof.

A promoter is usually located upstream of the coding sequence of the protein of interest. A preferable promoter which can be used according to the present disclosure is T7 promoter. An exemplary nucleotide sequence of T7 promoter is shown in SEQ ID NO: 8. In an embodiment, the regulatory sequence comprises a second promoter, which is located upstream of the polynucleotide according to the present disclosure. In a specific embodiment, the second promoter is a T7 promoter. In a specific embodiment, the T7 promoter comprises a nucleotide sequence of SEQ ID NO: 8.

A prokaryotic ribosome binding site is usually located between the promoter and the coding sequence of the protein of interest. Preferably, the prokaryotic ribosome binding site is located 5-10 bases upstream of the start codon. The prokaryotic ribosome binding site may be for example, a Shine-Dalgarno sequence. Preferably, the prokaryotic ribosome binding site is rich in AG. An exemplary nucleotide sequence of prokaryotic ribosome binding site is shown in SEQ ID NO: 9. In an embodiment, the regulatory sequence further comprises a second prokaryotic ribosome binding site, which is located between the second promoter as described above and the polynucleotide according to the present disclosure. In a specific embodiment, the second prokaryotic ribosome binding site comprises a nucleotide sequence of SEQ ID NO: 9.

The regulatory element of the Lac operon may be, for example, the promoter sequences and coding sequence of Lac operator (Lac operator, LacO), and Lac inhibitor (LacI). In a preferable embodiment, the expression cassette according to the present disclosure comprises a Lac operator, which is located between the promoter and the coding sequence of the protein of interest, and the promoter sequence and coding sequence of the Lac inhibitor are present in another expression cassette of the same vector. In other embodiments, the expression cassette according to the present disclosure comprises a Lac operator, which is located between the promoter and the coding sequence of the protein of interest, and the promoter sequence and coding sequence of the Lac inhibitor are present in a second vector. An exemplary nucleotide sequence of Lac operator is shown in SEQ ID NO: 10. In a further embodiment, the regulatory sequence further comprises a second Lac operator, which is located between the second promoter and the second prokaryotic ribosome binding site as described above. In a specific embodiment, the second Lac operator comprises a nucleotide sequence of SEQ ID NO: 10.

In a specific embodiment, the expression cassette or vector comprises, from 5'-end to 3'-end: (1) a second promoter; and (2) the polynucleotide according to the present disclosure. In a specific embodiment, the second promoter is a T7 promoter. In a specific embodiment, the T7 promoter comprises a nucleotide sequence of SEQ ID NO: 8.

In a specific embodiment, the expression cassette or vector comprises, from 5'-end to 3'-end: (1) a second promoter; (2) a second prokaryotic ribosome binding site; and (3) the polynucleotide according to the present disclosure. In a specific embodiment, the second promoter is a T7 promoter. In an embodiment, the T7 promoter comprises a nucleotide sequence of SEQ ID NO: 8. In a preferable embodiment, the second prokaryotic ribosome binding site sequence is rich in AG. In a specific embodiment, the second prokaryotic ribosome binding site comprises a nucleotide sequence of SEQ ID NO: 9.

In another specific embodiment, the expression cassette or vector comprises, from 5'-end to 3'-end: (1) a second promoter; (2) a second Lac operator; (3) a second prokaryotic ribosome binding site; and (4) the polynucleotide according to the present disclosure. In a specific embodiment, the second promoter is a T7 promoter. In a specific embodiment, the T7 promoter comprises a nucleotide sequence of SEQ ID NO: 8. In a preferable embodiment, the second prokaryotic ribosome binding site sequence is rich in AG, for example AAGGAG. In a specific embodiment, the second prokaryotic ribosome binding site comprises a nucleotide sequence of SEQ ID NO: 9. In another specific embodiment, the second Lac operator comprises a nucleotide sequence of SEQ ID NO: 10.

In the above-mentioned embodiments, the expression cassette or vector may further comprise a transcription terminator. The transcription terminator may be located downstream of the coding sequence of the protein of interest. Selection of the transcription terminator is coordinated with that of the promoter in the vector and the RNA polymerase contained in the host system. In an embodiment where the promoter is a T7 promoter and the transcription terminator may be a T7 transcription terminator. An exemplary nucleotide sequence of T7 transcription terminator is shown in SEQ ID NO: 11. In an embodiment, the second promoter is a T7 promoter and the transcription terminator is a T7 transcription terminator. In a specific embodiment, the T7 transcription terminator comprise a nucleotide sequence of SEQ ID NO: 11.

In an embodiment, the expression cassette or vector comprises, from 5'-end to 3'-end:
(1) a second T7 promoter (preferably having a nucleotide sequence of SEQ ID NO: 8);
(2) the polynucleotide according to the present disclosure (preferably having a nucleotide sequence of SEQ ID NO: 1 or SEQ ID NO: 2); and
(3) an optional T7 transcription terminator (preferably having a nucleotide sequence of SEQ ID NO: 11);

preferably, the expression cassette or vector further comprises a second prokaryotic ribosome binding site located between the second T7 promoter and the first nucleotide sequence (preferably, the second prokaryotic ribosome binding site has a nucleotide sequence of SEQ ID NO: 9);
more preferably, the expression cassette or vector further comprises a second Lac operator located between the second T7 promoter and the second prokaryotic ribosome binding site (preferably, the second Lac operator has a nucleotide sequence of SEQ ID NO: 10).

In a specific embodiment, the expression cassette or vector comprises, from 5'-end to 3'-end: (1) a second T7 promoter (preferably having a nucleotide sequence of SEQ ID NO: 8); (2) a second Lac operator (preferably having a nucleotide sequence of SEQ ID NO: 10); (3) a second prokaryotic ribosome binding site (preferably having a nucleotide sequence of SEQ ID NO: 9); (4) the polynucleotide according to the present disclosure (preferably having a nucleotide sequence of SEQ ID NO: 1 or SEQ ID NO: 2); and (5) a T7 transcription terminator (preferably having a nucleotide sequence of SEQ ID NO: 11).

The vector may also comprise a replication origin (e.g., pUC origin, pBR322 origin or pMB1 origin) and an antibiotic resistance gene (e.g., kanamycin resistance gene, carbenicillin resistance gene or ampicillin resistance gene) expression cassettes. The antibiotic resistance gene expression cassette may typically comprise the promoter and the coding sequence of the antibiotic resistance gene. The replication origin and antibiotic resistance gene expression cassettes which can be used in the present disclosure may be those known to those of skill in the art, such as those used in commercially available vectors.

The polynucleotide according to the present disclosure may be cloned into a commercially available vector. The vector that can be used in the present disclosure is preferably a plasmid vector. Preferably, a commercially available plasmid vector may comprise a T7 promoter, a prokaryotic ribosome binding site and a T7 transcription terminator, which may direct the expression of the polynucleotide according to the present disclosure in a host cell expressing T7 polymerase. More preferably, a commercially available plasmid vector may further comprise the regulatory element of Lac operon. Such a vector may be, for example, pET series vector (Novagen). In some embodiments, the vector according to the present disclosure may be derived from a vector selected from the group consisting of pET-3a-d, pET-9a-d(+), pET-11a-d, pET-14b, pET-21a-d(+), pET-23a-d(+), pET-24a-d(+), pET-30a-c(+), pET-28a-c(+), pET31b(+), pET32a-c(+), p33b, pET-41a-c(+), pET-42a-c(+). A phage vector and phagemid vector are also contemplated. In a specific embodiment, the vector according to the present disclosure is derived from pET-24a(+) and comprises a nucleotide sequence of SEQ ID NO: 1 or SEQ ID NO: 2.

### Host Cells

In another aspect, provided is a host cell, comprising the polynucleotide, the expression cassette or vector according to the present disclosure.

The expression cassette or vector according to the present disclosure may be introduced into a suitable host cell for the expression of toluene dioxygenase. Host cells which can be used in the present disclosure are commercially available. Preferably, the host cell is *E. coli.* For a host cell for protein expression, *E. coli* is more preferable. More preferably, the chromosome of the *E. coli* is integrated with a λ phage DE3 region (which contains the coding sequence of the T7 phage RNA polymerase), enabling *E. coli* to express the T7 RNA polymerase. More preferably, the expression of T7 RNA polymerase in *E. coli* is inducible. For example, the expression of T7 RNA polymerase in *E. coli* may be induced with IPTG, by placing the T7 RNA polymerase under the control of Lac operon. In an embodiment, the host cell is selected from the group consisting of *E. coli* BL21(DE3), BL21-Gold (DE3), BL21-Gold (DE3)pLysS, Rosetta (DE3), Origami^{™} 2 (DE3), OrigamiB (DE3), Origami^{™} B (DE3)pLysS, Rosetta-gami B (DE3) and Lemo21 (DE3). In a specific embodiment, the host cell is *E. coli* BL21(DE3), which is transformed with an expression vector of toluene dioxygenase, wherein the expression vector of toluene dioxygenase is derived from pET-24a(+) and comprises a nucleotide sequence of SEQ ID NO: 1 or SEQ ID NO: 2.

The vector according to the present disclosure may be introduced into a host cell using conventional transformation techniques known in the art. Such techniques include but are not limited to calcium phosphate co-precipitation, calcium chloride co-precipitation, heat shock, recombinant phage transduction, electroporation, triparental hybridization (see, for example Sambrook, J. et al., Molecular Cloning: A Laboratory Manual, 2nd ed., Cold Spring Harbor Laboratory Press, Cold Spring Harbor, N.Y, 1989).

### Process for Preparing Toluene Dioxygenase

In another aspect, provided is a process for preparing a toluene dioxygenase, comprising culturing the host cell according to the present disclosure under conditions suitable for the expression of the expression cassette or vector according to the present disclosure. In some embodiments, the process further comprises lysing the host cell expressing the toluene dioxygenase to give a lysate comprising the toluene dioxygenase. In another embodiment, the process further comprises purifying the toluene dioxygenase.

In a specific embodiment, the host cell is *E. coli* BL21(DE3) transformed with the vector according to the present disclosure.

In a specific embodiment, culturing the host cell under conditions suitable for the expression of the expression cassette or vector comprises:
(1) culturing *E. coli* BL21(DE3) transformed with the vector according to the present disclosure using an inorganic salt medium under conditions of temperature at about 30-37°C, pH of about 6.8-7.3 and dissolved oxygen of ≥20%;
(2) adding 66% glucose solution at a rate of 12-18 mL/hr/L for replenishment upon sudden rise in dissolved oxygen; and
(3) at OD600 of about 30-50, lowering the temperature to 20-30°C and adding isopropyl-β-D-thiogalactoside at a final concentration of 0.1-1 mM for inducible expression.

In a specific embodiment, the inorganic salt medium comprises 6-10 g/L KH₂PO₄•3H₂O, 11-15 g/L K₂HPO₄, 1-3 g/L (NH₄)₂SO₄, 1-3 g/L MgSO₄•7H₂O, 1-3 g/L trisodium citrate dihydrate, 2-5 g/L soybean peptone, 5-10 g/L glucose monohydrate, 0.1-1 g/L GPE, 10-20 mL/L trace element solution, which comprises: 10-20 g/L citric acid monohydrate, 1-5 g/L FeCl₃•6H₂O, 0.5-1 g/L ZnSO₄•7H₂O, 0.1-0.5 g/L CoCl₂•6H₂O, 0.1-0.5 g/L CuSO₄•5H₂O, 0.1-0.5 g/L H₃BO₃, 1-5 g/L MnSO₄•H₂O and 1-5 g/L CaCl₂•2H₂O.

### Processes for Preparing Cyclohexadiene o-diol compounds

In another aspect, provided is a process for preparing a compound of formula (2), 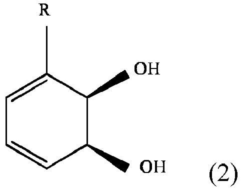 the process comprising,
(1) culturing the host cell according to the present disclosure under conditions suitable for the expression of the expression cassette or vector;
(2) adding a compound of formula (1) as substrate to the culture medium containing the host cell of step (1) for reaction to give a fermentation broth containing the compound of formula (2); 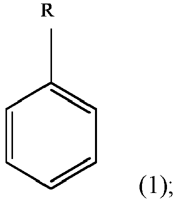 and
(3) recovering the compound of formula (2) from the fermentation broth of step (2);

wherein R is selected from the group consisting of halogen, -C₁₋₆ alkyl, -C₂₋₆ alkenyl, -C₁₋₆ alkyl -CN, -C₁₋₆ alkyl -O-C(=O)-C₁₋₆ alkyl, -C₃₋₈ cycloalkyl, -C₃₋₈ cycloalkenyl, and -C₆₋₁₀ aryl, wherein the C₁₋₆ alkyl, -C₂₋₆ alkenyl, -C₁₋₆ alkyl -CN, -C₁₋₆ alkyl -O-C(=O)-C₁₋₆ alkyl, -C₃₋₈cycloalkyl, -C₃₋₈ cycloalkenyl, and -C₆₋₁₀ aryl are optionally substituted with one or more halogen;
preferably, R is selected from the group consisting of halogen, methyl, -CH=CH₂, -CH=CHBr, -CH₂CH₂Br, -CH₂CH₂CN, -CH₂CH₂N₃, -CH₂CH₂-O-C(=O)-CH₃, -CH₂-O-C(=O)-CH₃, cyclohexyl, cyclohexenyl and phenyl.

In a specific embodiment, R is methyl, the compound of formula (1) is toluene and the compound of formula (2) is cis-(1S,2R)-3-methyl-3,5-cyclohexadiene-1,2-diol. In a specific embodiment, R is I, the compound of formula (1) is iodobenzene and the compound of formula (2) is cis-(1S,2R)-3-Iodo-3,5-cyclohexadiene-1,2-diol. In another specific embodiment, R is -CH₂-O-C(=O)-CH₃, the compound of formula (1) is benzyl acetate and the compound of formula (2) is cis-(1S,2R)-3-acetoxymethyl-3,5-cyclohexadiene-1,2-diol.

In a specific embodiment, the host cell is *E. oli* BL21(DE3), which is transformed with an expression vector of toluene dioxygenase, wherein the expression vector of toluene dioxygenase is derived from pET-24a(+) and comprises a nucleotide sequence of SEQ ID NO: 1 or SEQ ID NO: 2.

In a specific embodiment, step (1) of the above process comprises,
(1-1) culturing *E. coli* BL21(DE3) transformed with the vector according to the present disclosure using an inorganic salt medium under conditions of temperature at about 30-37°C, pH of about 6.8-7.3 and dissolved oxygen of ≥20%;
(1-2) adding 66% glucose solution at a rate of 12-18 mL/hr/L for replenishment upon sudden rise in dissolved oxygen; and
(1-3) at OD600 of about 30-50, lowering the temperature to 20-30°C and adding isopropyl-β-D-thiogalactoside (IPTG) at a final concentration of 0.1-1 mM for inducible expression.

In an embodiment, step (2) of the above process comprises, after initiation of IPTG-inducible expression, adding the compound of formula (1) to the fermenter in a flow-through manner, reducing the 66% glucose replenishment rate to 6-12 mL/hr/L until the end of fermentation. In an embodiment, step (2) further comprises detecting the product concentration using HPLC and stopping the reaction when the product concentration is no longer increasing.

In an embodiment, step (3) of the above process comprises, (3-1) collecting the supernatant of the fermentation broth by centrifugation; and (3-2) obtaining the product of the compound of formula (2) from the fermentation supernatant.

In a specific embodiment, step (3) comprises,
(3-1) Adjusting pH of the fermentation broth to 7.5-9.0 and collecting the fermentation broth supernatant by centrifugation;
(3-2) Adding dichloromethane or ethyl acetate to the supernatant of the fermentation broth from step (3-1) and extracting to give an aqueous phase and an organic phase containing the product; optionally, adding dichloromethane or ethyl acetate to the aqueous phase and extracting again to combine the organic phases;
(3-3) Concentrating the organic phase of step (3-2) under reduced pressure, adding appropriate amount of anhydrous sodium sulfate, and filtering to give the crude product;
(3-4) Continuing concentrating the crude product of step (3-3), adding petroleum ether for slurrying until the oil becomes solid; and
(3-5) adding methyl tert-butyl ether to the solid from step (3-4) for slurrying, and drying at 30-50°C under reduced pressure to give the product of the compound of formula (2).

In a specific embodiment, the compound of formula (1) is benzyl acetate, the compound of formula (2) is cis-(1S,2R)-3-acetoxymethyl-3,5-cyclohexadiene-1,2-diol.

Accordingly, in another aspect, provided is use of the polynucleotide, the expression cassette or vector, or the host cell according to the present disclosure for preparing a compound of formula (2).

The compound of formula (2) (particularly cis-(1S,2R)-3-acetoxymethyl-3,5-cyclohexadiene-1,2-diol and cis-(1S,2R)-3-Iodo-3,5-cyclohexadiene-1,2-diol) may be used as intermediates to prepare Tetrodotoxin (see, e.g., Baidilov, D. et al., Angew.Chem. Int. Ed. 2018, 57, 10994-10998). Accordingly, in another aspect, provided is also use of the polynucleotide, the expression cassette or vector, or the host cell according to the present disclosure for preparing Tetrodotoxin.

### Beneficial Effects

Provided is a polynucleotide, comprising coding sequences of toluene dioxygenase of *Pseudomonas putida* F1 strain (i.e., TodC1, TodC2, TodB and TodA). Provided is also an expression cassette and vector, comprising the polynucleotide according to the present disclosure. The polynucleotide, the expression cassette and vector according to the present disclosure are suitable for expressing the toluene dioxygenase in *E. coli.*

The expression system in the prior art (e.g., Baidilov, D. et al., Angew.Chem. Int. Ed. 2018, 57, 10994-10998) is pDTG601 vector and JM109 strain. The pDTG601 vector uses tac promoter, and the spacer sequences between the sequences encoding TodC1C2BA are directly derived from the genome of *Pseudomonas putida* F1 strain (GenBank accession number: J04996.1, nucleotides at positions 620-4210; sequence shown in SEQ ID NO: 12).

In contrast, the expression cassette or vector according to the present disclosure uses strong T7 promoter, *E. coli* preferred codons are used in the coding sequences of TodC1C2BA, and the spacer sequences between the coding sequences are replaced with a sequence containing T7 promoter and/or *E. coli* ribosome binding site. Constructed with the polynucleotide according to the present disclosure, the vector and recombinant strain are able to achieve the beneficial effect of efficient expression of toluene dioxygenase system in *E*. *coli.*

The polynucleotide, expression cassette, vector and re recombinant strain may be used to prepare cyclohexadiene *o*-diol compounds (e.g., cis-(1S,2R)-3-acetoxymethyl-3,5-cyclohexadiene-1,2-diol), with the product concentration of 2.1-7.8 g/L, e.g., 2.1, 2.3 and 7.8 g/L, which is higher than that of prior art as 1.7-2.1 g/L.

The expression system according to the present disclosure is capable of continuous production with high efficiency on a large scale, providing a novel option for the total synthesis on a large scale of complex molecules such as Tetrodotoxin with chiral cis-dihydrodiol compounds as precursors, making it possible to conduct clinical trials of Tetrodotoxin as a regulatory drug candidate, and being the foundation for the manufacture of Tetrodotoxin as a c-GMP active pharmaceutical ingredient for human analgesic and detoxification drug candidates.

### Examples

### Materials

Unless otherwise indicated, the instruments and reagents used herein are commercially available.

LB liquid medium: 10 g/L tryptone, 5 g/L yeast extract, 10 g/L NaCl.

TB liquid medium: 12 g/L tryptone, 24 g/L yeast extract, 4 mL/L glycerin, 2.3 g/L KH₂PO₄, 16.4 g/L K₂HPO₄•3H₂O.

Trace element solution: 14.4 g/L citric acid monohydrate, 3.0 g/L FeCl₃•6H₂O, 0.9 g/L ZnSO₄•7H₂O, 0.2 g/L CoCl₂•6H₂O, 0.2 g/L CuSO₄•5H₂O, 0.2 g/L H₃BO₃, 1.1 g/L MnSO₄•H₂O, 1.2 g/L CaCl₂•2H₂O.

Inorganic salt medium: 7.9 g/L KH₂PO₄•3H₂O, 13.0 g/L K₂HPO₄, 2.1 g/L (NH₄)₂SO₄, 1.7 g/L MgSO₄•7H₂O, 1.1 g/L trisodium citrate dihydrate, 2.5 g/L soybean peptone, 7.5 g/L glucose monohydrate, 0.5 g/L GPE, 10 mL/L trace element solution.

66% glucose solution: 660 g/L glucose monohydrate.

### Example 1: Construction of a strain expressing the dioxygenase system TodC1C2BA

Based on the TodC1C2BA encoding gene in *Pseudomonas putida* F1 strain (GenBank accession number: J04996.1, nucleotides at positions 620-4210 (SEQ ID NO: 12)), two polynucleotides suitable for expressing TodC1/TodC2/TodB/TodA proteins in *E. coli* system were designed, having the nucleotide sequences as shown in SEQ ID NO: 1 and SEQ ID NO: 2, respectively.

Compared to the nucleotide sequence of SEQ ID NO: 12, the polynucleotide according to the present disclosure has the following features:
(1) The coding sequences of TodC1, TodC2, TodB and TodA use *E. coli* preferred codons.
(2) The sequence of 110 nucleotides (first spacer sequence, S1) between the stop codon TGA for TodC1 and the start codon ATG for TodC2 in SEQ ID NO: 12 is replaced with the sequence of SEQ ID NO: 3. SEQ ID NO: 3 comprises, 5'-end to 3'-end, T7 promoter (SEQ ID NO: 8), Lac operator (LacO) (SEQ ID NO: 10) and prokaryotic ribosome binding site (rbs) sequence (SEQ ID NO: 9).
(3) The sequence between the stop codon TAG for TodC2 and the start codon ATG for TodB in SEQ ID NO: 12 (second spacer sequence, S2) GTGATGTC (nucleotides at positions 2028-2035 in SEQ ID NO: 1) remains unchanged at the corresponding positions in SEQ ID NO: 1, while is replaced with GAAGGAGATATACC (nucleotides at positions 2028-2041 in SEQ ID NO: 2, corresponding to SEQ ID NO: 9) at the corresponding positions in SEQ ID NO: 2.
(4) The stop codon TAA for TodB and the start codon ATG for TodA are codon nested sequence TAATG, consistent with SEQ ID NO: 12.

The polynucleotides containing the coding sequence of toluene dioxygenase (SEQ ID NO: 1 and SEQ ID NO: 2) were synthesized by GENEWIZ Suzhou Biotechnology Co. Ltd with a nucleotide sequence cat and the cleavage site ggatcc for the restriction endonuclease BamHI added at the 5'-end and 3'-end, respectively and then were cloned into the NdeI-BamHI sites of pET-24a(+) (Novagen) to give the expression plasmids pET-24a-SEQ1 and pET-24a-SEQ2, respectively (Fig. 1A and Fig. 1B). The expression plasmids were transformed into *E. coli* BL21(DE3) competent cells (Novagen) to give recombinant strains BL21(DE3)(pET-24a-SEQ1) and BL21(DE3)(pET-24a-SEQ2) expressing the toluene dioxygenase TodC1C2BA.

The polynucleotide with a sequence as shown in SEQ ID NO: 13 was designed. The coding sequences of TodC1, TodC2, TodB and TodA in SEQ ID NO: 13 use *E. coli* preferred codons and are identical to the corresponding sequences in SEQ ID NO: 1 and SEQ ID NO: 2, but the two spacer sequences S1 and S2 are consistent with the corresponding sequences in SEQ ID NO: 12. As described above, the expression plasmids pET-24a-SEQ12 and pET-24a-SEQ13 were constructed based on SEQ ID NO: 12 and SEQ ID NO: 13, respectively. Recombinant strains BL21(DE3)(pET-24a-SEQ12) and BL21(DE3)(pET-24a-SEQ13) were prepared by transforming pET-24a-SEQ12 and pET-24a-SEQ13 into BL21(DE3).

Figure 1C detailed the design differences among the coding sequences and spacer sequences in the four sequences as shown in SEQ ID NOs: 12, 13, 1 and 2.

### Example 2: Detection of protein levels in toluene dioxygenase TodC1C2BA expressing strains

Single colonies of the recombinant strains were seeded in the LB liquid medium (containing 50 µg/mL kanamycin) and grown for 16 hr at 37°C to obtain the seed solution. The seed solution was inoculated into the LB liquid medium (containing 50 µg/mL kanamycin) at 1% and incubated in the shaker at 37°C until OD600 = 0.5-1.0. The shaker temperature was lowered to 25°C and isopropyl-β-D-thiogalactoside (IPTG) at a final concentration of 0.1 mM was added for induction of expression. The shaker culture was continued until 24 hr. The fermentation broth was centrifuged at 4°C for 15 min at 8000 rpm to collect the bacteria. The bacteria were washed once with the binding buffer (50 mM Tris-HCl (pH 8.0), 0.5 M NaCl) and resuspended in the binding buffer (a small amount of bacteria solution was kept as whole cell (T) sample), and the suspension was sonicated in an ice-water bath. The lysed bacterial was centrifuged at 4°C, 12000 rpm for 20 min. The lysate supernatant (S) and precipitate (P) were collected respectively for protein sample preparation and subjected to SDS-PAGE protein gel electrophoresis analysis.

As shown in Fig. 2, the empty vector strain BL21(DE3)(pET-24a) showed no expression of the target protein.

In strain BL21(DE3)(pET-24a-SEQ12), only TodC1 was clearly expressed. Comparing samples 12T, 12S and 12P in Fig. 2, it can be seen that most of the expressed TodC1 protein was present in insoluble form in the inclusion body precipitate. In strain BL21(DE3)(pET-24a-SEQ13), only TodC1 was clearly expressed. Compared with samples 13T, 13S and 13P in Fig. 2, the expressed TodC1 protein was only partially soluble. It was shown that codon optimization of the target protein was very important for protein expression and solubility.

In strains BL21(DE3)(pET-24a-SEQ1) and BL21(DE3)(pET-24a-SEQ2), TodC1 and TodC2 proteins were clearly soluble, and there was no significant difference between the two strains in terms of protein expression levels. Compared with BL21(DE3)(pET-24a-SEQ13), the soluble expression of TodC2 protein was significantly increased in BL21(DE3)(pET-24a-SEQ1) and BL21(DE3)(pET-24a-SEQ2), indicating that the design of the first spacer sequence was very important for the expression of downstream proteins (at least for TodC2).

In addition, there were no bands for significant expression of TodB and TodA proteins in the four strains, which may be due to their coding sequences being located at more downstream positions in the expression cassette.

### Example 3: Strain BL21(DE3)(pET-24a-SEQ1) for use in preparation of cis-(1S,2R)-3-acetoxymethyl-3,5-cyclohexadiene-1,2-diol(CAS:131043-51-1)

Single colonies of strain BL21(DE3)(pET-24a-SEQ1) were seeded in the TB liquid medium (containing 50 µg/mL kanamycin) and grown for 16 hr at 37°C to give the seed solution. The seed solution was inoculated into a fermenter pre-filled with 25 L of inorganic salt medium (containing 50 µg/mL kanamycin) at 1% and incubated at 37°C. The pH was controlled at about 7.0 with aqueous ammonia, and the dissolved oxygen was controlled at ≥20% by adjusting the tank pressure, rotation speed and aeration. At about the fourth to fifth hr, the dissolved oxygen suddenly increased (indicating that the original carbon source was depleted), and the 66% glucose solution replenishment was initiated, with the replenishment rate set at 15 mL/hr/L. At about the eighth to ninth hr, when the OD600 was about 40, the temperature was lowered to 25°C and IPTG at a final concentration of 0.1 mM was added for induction of expression. At about the nineteenth to twenty-second hr, 90 g of the substrate benzyl acetate was added to the fermenter in a uniform flow rate over 3 hr, and the 66% glucose replenishment rate was reduced to 12 mL/hr/L until the end of fermentation. The conversion was stopped when the product no longer increased as detected by HPLC, and the product concentration was about 2.1 g/L. The HPLC chromatograph of the conversion process is shown in Figure 3.

The HPLC method: Agilent Liquid Chromatograph 1260 system; column, Agilent ZORBAX Eclipse XDB-C18 5µm (4.6 mm×250 mm); mobile phase: (A: methanol, B: water); flow rate: 1 mL/min; detection wavelength: 270 nm; column temperature: 30 °C; injection volume: 10 µL. HPLC gradient conditions:

| Time (min) | %A | %B | Note |
|---|---|---|---|
| 0 | 20 | 80 | Start of data collection |
| 20 | 20 | 80 | |
| 20.5 | 80 | 20 | |
| 27.5 | 80 | 20 | |
| 28 | 20 | 80 | End of data collection |
| 35 | 20 | 80 | |

Sample preparation: The fermentation broth was centrifuged at 12000 rpm for 10 min, the supernatant was diluted 5-10 times with methanol, mixed and centrifuged at 12000 rpm for 10 min, and the supernatant was loaded in the liquid phase.

After the fermentation transformation was completed, the pH of the fermentation broth was adjusted to 8.0 with 5 N NaOH. The fermentation broth supernatant was collected by centrifugation at 8000 rpm for 10 min, as about 30 L. 60 L of dichloromethane was added to the fermentation broth supernatant, which was stirred, and the organic phase was collected after standing for layering. 60 L of dichloromethane was added to the upper aqueous phase for extraction again and the organic phases were combined. The organic phase was concentrated to 1 L at 40°C under reduced pressure, and a brown clear solution was obtained by adding appropriate amount of anhydrous sodium sulfate and filtration. The clear solution was further concentrated and dried, and then slurried with the addition of petroleum ether until the oil became solid. The supernatant was discarded. The solid was then slurried with methyl tert-butyl ether and dried at 30°C under reduced pressure to give 45.3 g of product as off-white solid. The NMR spectrum (Bruker 400 MHz) of the product cis-(1S,2R)-3-acetoxymethyl-3,5-cyclohexadiene-1,2-diol (CAS:131043-51-1) was shown in Fig. 4.

### Example 4: Strain BL21(DE3)(pET-24a-SEQ2) for use in preparation of cis-(1S,2R)-3-acetoxymethyl-3,5-cyclohexadiene-1,2-diol(CAS:131043-51-1)

Single colonies of strain BL21(DE3)(pET-24a-SEQ2) were seeded in the TB liquid medium (containing 50 µg/mL kanamycin) and grown for 16 hr at 37°C to give the seed solution. The seed solution was inoculated into a fermenter pre-filled with 25 L of inorganic salt medium (containing 50 µg/mL kanamycin) at 1% and incubated at 37°C. The pH was controlled at about 7.0 with aqueous ammonia, and the dissolved oxygen was controlled at ≥20% by adjusting the tank pressure, rotation speed and aeration. At about the fourth to fifth hr, the dissolved oxygen suddenly increased (indicating that the original carbon source was depleted), and the 66% glucose solution replenishment was initiated, with the replenishment rate set at 15 mL/hr/L. At about the eighth to ninth hr, when the OD600 was about 40, the temperature was lowered to 25°C and IPTG at a final concentration of 0.1 mM was added for induction of expression. At about the nineteenth to twenty-second hr, 90 g of the substrate benzyl acetate was added to the fermenter in a uniform flow rate over 3 hr, and the 66% glucose replenishment rate was reduced to 12 mL/hr/L until the end of fermentation. The conversion was stopped when the product no longer increased as detected by HPLC, and the product concentration was about 2.3 g/L.

After the fermentation transformation was completed, the pH of the fermentation broth was adjusted to 8.0 with 5 N NaOH. The fermentation broth supernatant was collected by centrifugation at 8000 rpm for 10 min, as about 30 L. 30 L of ethyl acetate was added to the fermentation broth supernatant, which was stirred, and the organic phase was collected after standing for layering. 30 L of ethyl acetate was added to the upper aqueous phase for extraction again and the organic phases were combined. The organic phase was concentrated to 1 L at 40°C under reduced pressure, and a brown clear solution was obtained by adding appropriate amount of anhydrous sodium sulfate and filtration. The clear solution was further concentrated and dried, and then slurried with the addition of petroleum ether until the oil became solid. The supernatant was discarded. The solid was then slurried with methyl tert-butyl ether and dried at 30°C under reduced pressure to give about 50 g of product as off-white solid.

### Example 5: Strains BL21(DE3)(pET-24a-SEQ12) and BL21(DE3)(pET-24a-SEQ13) for use in biotransformation of cis-(1S,2R)-3-acetoxymethyl-3,5-cyclohexadiene-1,2-diol(CAS:131043-51-1)

Single colonies of strain BL21(DE3)(pET-24a-SEQ12) were seeded in the TB liquid medium (containing 50 µg/mL kanamycin) and grown for 16 hr at 37°C to give the seed solution. The seed solution was inoculated into a fermenter pre-filled with 25 L of inorganic salt medium (containing 50 µg/mL kanamycin) at 1% and incubated at 37°C. The pH was controlled at about 7.0 with aqueous ammonia, and the dissolved oxygen was controlled at ≥20% by adjusting the tank pressure, rotation speed and aeration. At about the fourth to fifth hr, the dissolved oxygen suddenly increased (indicating that the original carbon source was depleted), and the 66% glucose solution replenishment was initiated, with the replenishment rate set at 15 mL/hr/L. At about the eighth to ninth hr, when the OD600 was about 40, the temperature was lowered to 25°C and IPTG at a final concentration of 0.1 mM was added for induction of expression. At about the nineteenth to twenty-second hr, 90 g of the substrate benzyl acetate was added to the fermenter in a uniform flow rate over 3 hr, and the 66% glucose replenishment rate was reduced to 12 mL/hr/L until the end of fermentation. The concentration of the product was about 0.7 g/L as detected by HPLC, with a large amount of substrate remained.

The strain BL21(DE3)(pET-24a-SEQ13) was subjected to biotransformation as described above, and the product concentration was about 0.9 g/L as detected by HPLC, with a large amount of substrate remained.

### Example 6: Strain BL21(DE3)(pET-24a-SEQ2) for use in preparation of cis-(1S,2R)-3-acetoxymethyl-3,5-cyclohexadiene-1,2-diol(CAS:131043-51-1) in hundred grams scale

Single colonies of strain BL21(DE3)(pET-24a-SEQ2) were seeded in the TB liquid medium (containing 50 µg/mL kanamycin) and grown for 16 hr at 37°C to give the seed solution. The seed solution was inoculated into a fermenter pre-filled with 50 L of inorganic salt medium (containing 50 µg/mL kanamycin) at 1% and incubated at 37°C. The pH was controlled at about 7.0 with aqueous ammonia, and the dissolved oxygen was controlled at ≥20% by adjusting the tank pressure, rotation speed and aeration. At about the fourth to fifth hr, the dissolved oxygen suddenly increased (indicating that the original carbon source was depleted), and the 66% glucose solution replenishment was initiated, with the replenishment rate set at 15 mL/hr/L. At about the eighth to ninth hr, when the OD600 was about 40, the temperature was lowered to 25°C and IPTG at a final concentration of 0.1 mM was added for induction of expression. At about the fifteenth to twenty-second hr, 700 g of the substrate benzyl acetate was added to the fermenter in a uniform flow rate over 7 hr, and the 66% glucose replenishment rate was reduced to 12 mL/hr/L until the end of fermentation. The conversion was stopped when the product no longer increased as detected by HPLC, and the concentration of the product was about 7.8 g/L.

After the fermentation transformation was completed, the pH of the fermentation broth was adjusted to 8.0 with 5 N NaOH. The supernatant of the fermentation broth was collected by centrifugation in a continuous flow tube centrifuge, as about 60 L. 60 L of ethyl acetate was added to the fermentation broth supernatant, which was stirred, and the organic phase was collected after standing for layering. 60 L of ethyl acetate was added to the upper aqueous phase for extraction again and the organic phases were combined. The organic phase was concentrated to 10 L at 40°C under reduced pressure, and a brown clear solution was obtained by adding appropriate amount of anhydrous sodium sulfate and filtration. The clear solution was further concentrated and dried, and then slurried with the addition of petroleum ether until the oil became solid. The supernatant was discarded. The solid was then slurried with methyl tert-butyl ether and dried at 30°C under reduced pressure to give about 351 g of product as off-white solid.

Although the present invention has been disclosed as above with detailed embodiments, they are not intended to limit the same. Those skilled in the art can make various changes and modifications without departing from the spirit and scope of the invention. Accordingly, the scope of protection of the invention should be defined by the claims.

### Sequence Listing

SEQ ID NO: 1: polynucleotide comprising coding sequences of toluene dioxygenase 
atgaaccagaccgacacctctccgatccgtctgcgtcgttcttggaacacctctgaaatcgaagctctgttcgacgaacacgctggtcgt
atcgacccgcgtatctacaccgacgaagacctgtaccagctggaactcgaacgtgtcttcgcgcgttcttggctgctgctgggtcacga
aacccagatccgtaaaccgggtgactacatcaccacctacatgggtgaagacccggttgttgttcgtcagaaagacgcttctatcgc
tgttttcctgaaccagtgccgtcaccgtggtatgcgtatctgccgtgctgacgctggtaacgctaaagctttcacctgctcttaccacggtt
gggcttacgacaccgctggtaacctggttaacgttccgtacgaagctgaatctttcgcttgcctgaacaaaaaagaatggtctccgctga
aagctcgtgttgaaacctacaaaggtctgatcttcgctaactgggacgaaaacgctgttgacctggacacctacctgggtgaagctaaat
tctacatggaccacatgctggaccgtaccgaagctggtaccgaagctatcccgggtgttcagaaatgggttatcccgtgcaactggaaa
ttcgctgctgaacagttctgctctgacatgtaccacgctggtaccacctctcacctgtctggtatcctggctggtctgccggaagacctgg
aaatggctgacctggctccgccgaccgttggtaaacagtaccgtgcttcttggggtggtcacggttctggtttctacgttggtgacccga
acctgatgctggctatcatgggtccgaaagttacctcttactggaccgaaggtccggcttctgaaaaagctgctgaacgtctgggttctgt
tgaacgtggttctaaactgatggttgaacacatgaccgttttcccgacctgctctttcctgccgggtatcaacaccgttcgtacctggcacc
cgcgtggtccgaacgaggttgaagtatgggcgttcaccgttgttgacgctgacgctccggacgacatcaaagaagaattccgtcgtca
gaccctgcgtaccttctctgctggtggtgttttcgaacaggacgacggtgaaaactgggttgaaatccagcacatcctgcgtggtcacaa
agctcgttctcgtccgttcaacgctgaaatgtctatggaccagaccgttgacaacgacccggtttacccgggtcgtatctctaacaacgtt
tactctgaagaagctgctcgtggtctgtacgctcactggctgcgtatgatgacctctccggactgggacgctctgaaagctacccgttaat
cgagatctcgatcccgcgaaattaatacgactcactataggggaattgtgagcggataacaattcccctctagaaataattttgtttaacttt
aagaaggagatataccatgatcgactctgctaaccgtgctgacgttttcctgcgtaaaccggctccggttgctccggaactgcagcacg
aagttgaacagttctactactgggaagctaaactgctgaacgaccgtcgtttcgaagaatggttcgctctgctggctgaagacatccact
acttcatgccgatccgtaccacccgtatcatgcgtgactctcgtctggaatactctggttctcgtgaatacgctcacttcgacgacgacgct
 
accatgatgaaaggtcgtctgcgtaaaatcacctctgacgtttcttggtctgaaaacccggcttctcgtacccgtcacctggtttctaacgtt
atgatcgttggtgctgaagctgaaggtgaatacgaaatctcttctgctttcatcgtttaccgtaaccgtctggaacgtcagctggacatcttc
gctggtgaacgtcgtgacaccctgcgtcgtaacacctctgaagctggtttcgaaatcgttaaccgtaccatcctgatcgaccagtctacc
atcctggctaacaacctgtctttcttcttctaagtgatgtcatgacctggacctacatcctgcgtcagggtgacctgccgccgggtgaaatg
cagcgttacgaaggtggtccggaaccggttatggtttgcaacgttgacggtgaatttttcgctgttcaggacacctgcacccacggtgac
tgggctctgtctgacggttacctggacggtgacatcgttgaatgcaccctgcacttcggtaaattctgcgttcgtaccggtaaagttaaag
ctctgccggcttgcaaaccgatcaaagttttcccgatcaaagttgaaggtgacgaagttcacgttgacctggacaacggtgaactgaaat
aatggctacccacgttgctatcatcggtaacggcgttggtggcttcaccaccgctcaggctctgcgtgcggaaggcttcgaaggtcgta
tatctctgatcggtgacgaaccgcacctgccgtacgaccgtccgtctctgtctaaagctgttctggacggttctctggaacgtccgccgat
cctggctgaagctgactggtacggtgaagctcgtatcgacatgctgaccggtccggaagttaccgctctggacgttcagacccgtacc
atctctctggacgacggtaccaccctgtctgctgacgctatcgttatcgctaccggttctcgtgctcgtactatggctctgccgggttctca
gctgccgggtgttgttaccctgcgtacctacggtgacgttcaggttctgcgtgactcttggacctctgctacccgtctgctgatcgttggtg
gtggtctgatcggttgcgaagttgctaccaccgctcgtaaactgggtctgtctgttaccatcctggaagctggtgacgaactgctggttcg
tgttctgggtcgtcgtatcggtgcttggctgcgtggtctgctgaccgaactgggtgttcaggttgaactgggtaccggtgttgttggtttctc
tggtgaaggtcagctggaacaggttatggcttctgacggtcgttctttcgttgctgactctgctctgatctgcgttggtgctgaaccggctg
accagctggctcgtcaggctggtctggcttgcgaccgtggtgttatcgttgaccactgcggtgctaccctggctaaaggtgttttcgctgt
tggtgacgttgcttcttggccgctgcgtgctggtggtcgtcgttctctggaaacctacatgaacgctcagcgtcaggctgctgctgttgct
gctgctatcctgggtaaaaacgtttctgcgccgcagctgccggtgagctggaccgaaatcgctggtcaccgtatgcagatggctggtg
acatcgaaggtccgggtgacttcgtttctcgtggtatgccgggttctggtgctgctctgctgttccgtctgcaagaacgtcgtatccaggct
gttgttgctgttgacgctccgcgtgacttcgctctggctacccgtctggttgaagctcgtgctgctatcgaaccggctcgtctggctgacct
gtctaactctatgcgtgacttcgttcgtgctaacgaaggtgacctgacctaa
SEQ ID NO: 2: polynucleotide comprising coding sequences of toluene dioxygenase 
atgaaccagaccgacacctctccgatccgtctgcgtcgttcttggaacacctctgaaatcgaagctctgttcgacgaacacgctggtcgt
atcgacccgcgtatctacaccgacgaagacctgtaccagctggaactcgaacgtgtcttcgcgcgttcttggctgctgctgggtcacga
aacccagatccgtaaaccgggtgactacatcaccacctacatgggtgaagacccggttgttgttcgtcagaaagacgcttctatcgc
tgttttcctgaaccagtgccgtcaccgtggtatgcgtatctgccgtgctgacgctggtaacgctaaagctttcacctgctcttaccacggtt
gggcttacgacaccgctggtaacctggttaacgttccgtacgaagctgaatctttcgcttgcctgaacaaaaaagaatggtctccgctga
aagctcgtgttgaaacctacaaaggtctgatcttcgctaactgggacgaaaacgctgttgacctggacacctacctgggtgaagctaaat
tctacatggaccacatgctggaccgtaccgaagctggtaccgaagctatcccgggtgttcagaaatgggttatcccgtgcaactggaaa
ttcgctgctgaacagttctgctctgacatgtaccacgctggtaccacctctcacctgtctggtatcctggctggtctgccggaagacctgg
aaatggctgacctggctccgccgaccgttggtaaacagtaccgtgcttcttggggtggtcacggttctggtttctacgttggtgacccga
acctgatgctggctatcatgggtccgaaagttacctcttactggaccgaaggtccggcttctgaaaaagctgctgaacgtctgggttctgt
tgaacgtggttctaaactgatggttgaacacatgaccgttttcccgacctgctctttcctgccgggtatcaacaccgttcgtacctggcacc
cgcgtggtccgaacgaggttgaagtatgggcgttcaccgttgttgacgctgacgctccggacgacatcaaagaagaattccgtcgtca
gaccctgcgtaccttctctgctggtggtgttttcgaacaggacgacggtgaaaactgggttgaaatccagcacatcctgcgtggtcacaa
agctcgttctcgtccgttcaacgctgaaatgtctatggaccagaccgttgacaacgacccggtttacccgggtcgtatctctaacaacgtt
tactctgaagaagctgctcgtggtctgtacgctcactggctgcgtatgatgacctctccggactgggacgctctgaaagctacccgttaat
cgagatctcgatcccgcgaaattaatacgactcactataggggaattgtgagcggataacaattcccctctagaaataattttgtttaacttt
aagaaggagatataccatgatcgactctgctaaccgtgctgacgttttcctgcgtaaaccggctccggttgctccggaactgcagcacg
aagttgaacagttctactactgggaagctaaactgctgaacgaccgtcgtttcgaagaatggttcgctctgctggctgaagacatccact
 
acttcatgccgatccgtaccacccgtatcatgcgtgactctcgtctggaatactctggttctcgtgaatacgctcacttcgacgacgacgct
accatgatgaaaggtcgtctgcgtaaaatcacctctgacgtttcttggtctgaaaacccggcttctcgtacccgtcacctggtttctaacgtt
atgatcgttggtgctgaagctgaaggtgaatacgaaatctcttctgctttcatcgtttaccgtaaccgtctggaacgtcagctggacatcttc
gctggtgaacgtcgtgacaccctgcgtcgtaacacctctgaagctggtttcgaaatcgttaaccgtaccatcctgatcgaccagtctacc
atcctggctaacaacctgtctttcttcttctaagaaggagatataccatgacctggacctacatcctgcgtcagggtgacctgccgccggg
tgaaatgcagcgttacgaaggtggtccggaaccggttatggtttgcaacgttgacggtgaatttttcgctgttcaggacacctgcaccca
cggtgactgggctctgtctgacggttacctggacggtgacatcgttgaatgcaccctgcacttcggtaaattctgcgttcgtaccggtaaa
gttaaagctctgccggcttgcaaaccgatcaaagttttcccgatcaaagttgaaggtgacgaagttcacgttgacctggacaacggtgaa
ctgaaataatggctacccacgttgctatcatcggtaacggcgttggtggcttcaccaccgctcaggctctgcgtgcggaaggcttcgaa
ggtcgtatatctctgatcggtgacgaaccgcacctgccgtacgaccgtccgtctctgtctaaagctgttctggacggttctctggaacgtc
cgccgatcctggctgaagctgactggtacggtgaagctcgtatcgacatgctgaccggtccggaagttaccgctctggacgttcagac
ccgtaccatctctctggacgacggtaccaccctgtctgctgacgctatcgttatcgctaccggttctcgtgctcgtactatggctctgccgg
gttctcagctgccgggtgttgttaccctgcgtacctacggtgacgttcaggttctgcgtgactcttggacctctgctacccgtctgctgatc
gttggtggtggtctgatcggttgcgaagttgctaccaccgctcgtaaactgggtctgtctgttaccatcctggaagctggtgacgaactgc
tggttcgtgttctgggtcgtcgtatcggtgcttggctgcgtggtctgctgaccgaactgggtgttcaggttgaactgggtaccggtgttgtt
ggtttctctggtgaaggtcagctggaacaggttatggcttctgacggtcgttctttcgttgctgactctgctctgatctgcgttggtgctgaa
ccggctgaccagctggctcgtcaggctggtctggcttgcgaccgtggtgttatcgttgaccactgcggtgctaccctggctaaaggtgtt
ttcgctgttggtgacgttgcttcttggccgctgcgtgctggtggtcgtcgttctctggaaacctacatgaacgctcagcgtcaggctgctg
ctgttgctgctgctatcctgggtaaaaacgtttctgcgccgcagctgccggtgagctggaccgaaatcgctggtcaccgtatgcagatg
gctggtgacatcgaaggtccgggtgacttcgtttctcgtggtatgccgggttctggtgctgctctgctgttccgtctgcaagaacgtcgtat
ccaggctgttgttgctgttgacgctccgcgtgacttcgctctggctacccgtctggttgaagctcgtgctgctatcgaaccggctcgtctg
gctgacctgtctaactctatgcgtgacttcgttcgtgctaacgaaggtgacctgacctaa
SEQ ID NO: 3: first spacer sequence 
tcgagatctcgatcccgcgaaattaatacgactcactataggggaattgtgagcggataacaattcccctctagaaataattttgtttaactt
taagaaggagatatacc
SEQ ID NO: 4: TodC1 
MNQTDTSPIRLRRSWNTSEIEALFDEHAGRIDPRIYTDEDLYQLELERVFARSWLLLG
HETQIRKPGDYITTYMGEDPVVVVRQKDASIAVFLNQCRHRGMRICRADAGNAKAF
TCSYHGWAYDTAGNLVNVPYEAESFACLNKKEWSPLKARVETYKGLIFANWDENA
VDLDTYLGEAKFYMDHMLDRTEAGTEAIPGVQKWVIPCNWKFAAEQFCSDMYHAG
TTSHLSGILAGLPEDLEMADLAPPTVGKQYRASWGGHGSGFYVGDPNLMLAIMGPK
VTSYWTEGPASEKAAERLGSVERGSKLMVEHMTVFPTCSFLPGINTVRTWHPRGPNE
VEVWAFTVVDADAPDDIKEEFRRQTLRTFSAGGVFEQDDGENWVEIQHILRGHKARS
RPFNAEMSMDQTVDNDPVYPGRISNNVYSEEAARGLYAHWLRMMTSPDWDALKAT
R
SEQ ID NO: 5: TodC2 
MIDSANRADVFLRKPAPVAPELQHEVEQFYYWEAKLLNDRRFEEWFALLAEDIHYF
MPIRTTRIMRDSRLEYSGSREYAHFDDDATMMKGRLRKITSDVSWSENPASRTRHLV
 
SNVMIVGAEAEGEYEISSAFIVYRNRLERQLDIFAGERRDTLRRNTSEAGFEIVNRTILI
DQSTILANNLSFFF
SEQ ID NO: 6: TodB 
MTWTYILRQGDLPPGEMQRYEGGPEPVMVCNVDGEFFAVQDTCTHGDWALSDGYL
DGDIVECTLHFGKFCVRTGKVKALPACKPIKVFPIKVEGDEVHVDLDNGELK
SEQ ID NO: 7: TodA
SEQ ID NO: 8: T7 promoter
   taatacgactcactatagg
SEQ ID NO: 9: prokaryotic ribosome binding site
   gaaggagatatacc
SEQ ID NO: 10: Lac operator
   ttgtgagcggataacaa
SEQ ID NO: 11: T7 transcription terminator
   ctagcataaccccttggggcctctaaacgggtcttgaggggttttttg
SEQ ID NO: 12: nucleotides at positions 620-4210 in J04996.1 
atgaatcagaccgacacatcacctatcaggctgcgcaggagctggaacaccagcgagatagaagcgctctttgacgagcatgccgg
acgtatcgatccgcgcatttataccgatgaggatctgtaccaactcgaactggagcgtgtcttcgcccggtcctggctgctgttggggca
tgaaacccagattcgcaagccgggcgattacatcacgacctacatgggtgaagaccctgtcgtggtcgtccggcagaaagacgccag
cattgccgtgttcctgaaccagtgccgccaccgtggcatgcgcatctgccgcgcggatgccggaaacgcgaaggcgttcacttgcag
ctaccacgggtgggcttacgacaccgccggcaatcttgtcaatgtgccttacgaggccgaatccttcgcgtgcctgaacaagaaggaa
tggagcccgctgaaggcccgggtagaaacctacaagggcctgattttcgccaactgggatgagaacgctgtagacctcgacacgtat
ctgggcgaggcgaagttctacatggaccacatgctcgaccgcaccgaggccggcaccgaagcgatcccgggcgtgcagaagtgg
gtcattccctgtaactggaaattcgccgcagagcagttttgcagcgacatgtaccatgccgggacgacctcgcatctgtctggcatcctg
gcaggcctgccagaagaccttgaaatggccgaccttgctccgccgacagttggcaagcagtaccgtgcgtcatggggcggacatgg
aagtggcttctatgtcggcgaccccaatctgatgcttgccatcatggggccaaaggtcaccagctactggaccgaaggccccgcgtcg
gaaaaggcggccgaacgtctgggtagcgtggagcgcggctcgaaactcatggtcgagcacatgaccgtcttccccacgtgttccttcc
 
tcccaggtatcaatacggtccggacatggcatccgcgcgggccgaacgaggtcgaggtatgggcgtttacggtggtcgatgctgatg
ctcctgacgatatcaaggaagagttccggcgccagacgctgcgcaccttctctgccggtggcgtgttcgagcaggacgacggggag
aactgggtcgagatccagcacatcctgcgaggccacaaggcgcggagccgccctttcaatgccgagatgagcatggaccagaccgt
cgacaacgacccggtttaccccgggcggatcagcaacaacgtctacagcgaggaagctgcccgcgggctctatgcccattggctgc
ggatgatgacatcccccgactgggacgcgctgaaggcgacacgctgaatccagagacagcttgcgccacgcagtggcgccggcca
gaggccgcatttgacttcgacccaggttggatgcggtggaccttgtccatttgaaatctacaaggaacgaccatgattgattcagccaac
agagccgacgtctttctccgcaagccggcacccgtagcgcccgaactgcagcacgaagtcgagcagttctactattgggaggccaag
cttctcaacgatcgccgcttcgaggagtggttcgcgctgctcgcggaagacattcactacttcatgcccattcgcaccacgcggatcatg
cgggactcgcgccttgaatactcaggctcccgagagtacgcgcacttcgatgacgacgccacgatgatgaagggacgcttgcgcaa
gatcacgtccgacgtgagctggtccgagaaccccgcatcgcggacccggcatctcgtgagcaacgtgatgatcgtcggcgcagagg
cagaaggggagtacgaaatctcaagcgccttcattgtgtaccgcaatcgtctggagcggcagctcgacatctttgccggtgagcgtcg
cgatacgttgcgccgtaacacgagcgaggccgggttcgagatcgtcaatcggaccatcctgatcgaccagagcaccatcctggccaa
taacctcagtttcttcttctaggtgatgtcatgacttggacatacatattgcggcagggtgacctgccacccggtgagatgcagcgctacg
aaggcggcccggaacctgtgatggtctgcaacgtcgatggcgagttcttcgcggtgcaggatacctgcacgcatggggactgggcgt
tgtcggatggttacctggacggtgatattgtcgaatgcacgttgcatttcggcaagttctgcgtgcggaccgggaaggtgaaggcgctg
cctgcttgcaaacctatcaaggtattcccaatcaaggtcgaaggcgatgaagtgcacgtcgatctcgacaacggggagttgaagtgatg
gctacccatgtggcgatcatcggcaatggcgtgggtggcttcacgaccgcgcaggccctacgtgccgagggcttcgaggggagaat
ctcgctgattggggacgaaccgcatctcccctatgaccgaccatccttgtccaaggcggttctcgacggcagccttgagcggccgccc
atactggccgaggccgattggtacggcgaggcccgcatcgacatgctgaccggcccggaagtcactgcccttgatgtgcagacaag
gacgatcagtctggatgatggcaccacgctctctgcggacgccatcgtcatcgcgacgggcagtcgagcgcggacgatggcgttgc
ccggcagccaactgcccggcgtcgtaacgctgcgcacctacggtgacgtgcaggtattgcgcgatagttggacttccgcgacgcggc
tgctgattgtgggtggcggattgatcggctgcgaggtcgcgacgacggcgcgcaagctcggcctgtcggtcacgatcctggaggca
ggtgatgaactgctggtccgagtacttgggcggcgtatcggtgcctggctgcgcggcctgctgacagaacttggtgtgcaggtcgagt
tgggaacgggtgtcgtaggtttttctggtgagggccagctcgaacaagtcatggccagcgatgggcgcagcttcgtagccgatagcgc
actcatttgcgtcggcgcggagcccgcggatcaacttgcgcgtcaagcgggcttggcatgtgaccgcggcgtcattgtcgatcactgc
ggtgcgacgcttgccaaaggcgtattcgccgtcggagatgtggccagttggccgctgcgcgccggcggccggcgttcgctcgaaac
ctatatgaacgcgcagcgccaagccgccgcggtggctgcggccattctggggaaaaacgtatcggcaccgcaactgcccgtgtcct
ggacggagatcgctgggcatcgcatgcagatggcgggcgatatcgaaggacctggtgatttcgtctcgcgcggcatgcccggtagtg
gcgctgccctgttgttccgcctgcaggagcgaaggattcaggcggtcgtcgcggtcgatgcaccccgtgacttcgcgcttgcaacccg
attggtagaagcccgcgcggcaatcgagccagcacggctggcagatctttcaaacagtatgcgcgattttgttcgtgcgaatgaagga
gacctaacgtga
SEQ ID NO: 13: polynucleotide comprising coding sequences of toluene dioxygenase

atgaaccagaccgacacctctccgatccgtctgcgtcgttcttggaacacctctgaaatcgaagctctgttcgacgaacacgctggtcgt
atcgacccgcgtatctacaccgacgaagacctgtaccagctggaactcgaacgtgtcttcgcgcgttcttggctgctgctgggtcacga
aacccagatccgtaaaccgggtgactacatcaccacctacatgggtgaagacccggttgttgttcgtcagaaagacgcttctatcgc
tgttttcctgaaccagtgccgtcaccgtggtatgcgtatctgccgtgctgacgctggtaacgctaaagctttcacctgctcttaccacggtt
gggcttacgacaccgctggtaacctggttaacgttccgtacgaagctgaatctttcgcttgcctgaacaaaaaagaatggtctccgctga
aagctcgtgttgaaacctacaaaggtctgatcttcgctaactgggacgaaaacgctgttgacctggacacctacctgggtgaagctaaat
tctacatggaccacatgctggaccgtaccgaagctggtaccgaagctatcccgggtgttcagaaatgggttatcccgtgcaactggaaa
ttcgctgctgaacagttctgctctgacatgtaccacgctggtaccacctctcacctgtctggtatcctggctggtctgccggaagacctgg

aaatggctgacctggctccgccgaccgttggtaaacagtaccgtgcttcttggggtggtcacggttctggtttctacgttggtgacccga
acctgatgctggctatcatgggtccgaaagttacctcttactggaccgaaggtccggcttctgaaaaagctgctgaacgtctgggttctgt
tgaacgtggttctaaactgatggttgaacacatgaccgttttcccgacctgctctttcctgccgggtatcaacaccgttcgtacctggcacc
cgcgtggtccgaacgaggttgaagtatgggcgttcaccgttgttgacgctgacgctccggacgacatcaaagaagaattccgtcgtca
gaccctgcgtaccttctctgctggtggtgttttcgaacaggacgacggtgaaaactgggttgaaatccagcacatcctgcgtggtcacaa
agctcgttctcgtccgttcaacgctgaaatgtctatggaccagaccgttgacaacgacccggtttacccgggtcgtatctctaacaacgtt
tactctgaagaagctgctcgtggtctgtacgctcactggctgcgtatgatgacctctccggactgggacgctctgaaagctacccgttaa
tccagagacagcttgcgccacgcagtggcgccggccagaggccgcatttgacttcgacccaggttggatgcggtggaccttgtccatt
tgaaatctacaaggaacgaccatgatcgactctgctaaccgtgctgacgttttcctgcgtaaaccggctccggttgctccggaactgcag
cacgaagttgaacagttctactactgggaagctaaactgctgaacgaccgtcgtttcgaagaatggttcgctctgctggctgaagacatc
cactacttcatgccgatccgtaccacccgtatcatgcgtgactctcgtctggaatactctggttctcgtgaatacgctcacttcgacgacga
cgctaccatgatgaaaggtcgtctgcgtaaaatcacctctgacgtttcttggtctgaaaacccggcttctcgtacccgtcacctggtttcta
acgttatgatcgttggtgctgaagctgaaggtgaatacgaaatctcttctgctttcatcgtttaccgtaaccgtctggaacgtcagctggac
atcttcgctggtgaacgtcgtgacaccctgcgtcgtaacacctctgaagctggtttcgaaatcgttaaccgtaccatcctgatcgaccagt
ctaccatcctggctaacaacctgtctttcttcttctaagtgatgtcatgacctggacctacatcctgcgtcagggtgacctgccgccgggtg
aaatgcagcgttacgaaggtggtccggaaccggttatggtttgcaacgttgacggtgaatttttcgctgttcaggacacctgcacccacg
gtgactgggctctgtctgacggttacctggacggtgacatcgttgaatgcaccctgcacttcggtaaattctgcgttcgtaccggtaaagt
taaagctctgccggcttgcaaaccgatcaaagttttcccgatcaaagttgaaggtgacgaagttcacgttgacctggacaacggtgaact
gaaataatggctacccacgttgctatcatcggtaacggcgttggtggcttcaccaccgctcaggctctgcgtgcggaaggcttcgaagg
tcgtatatctctgatcggtgacgaaccgcacctgccgtacgaccgtccgtctctgtctaaagctgttctggacggttctctggaacgtccg
ccgatcctggctgaagctgactggtacggtgaagctcgtatcgacatgctgaccggtccggaagttaccgctctggacgttcagaccc
gtaccatctctctggacgacggtaccaccctgtctgctgacgctatcgttatcgctaccggttctcgtgctcgtactatggctctgccgggt
tctcagctgccgggtgttgttaccctgcgtacctacggtgacgttcaggttctgcgtgactcttggacctctgctacccgtctgctgatcgtt
ggtggtggtctgatcggttgcgaagttgctaccaccgctcgtaaactgggtctgtctgttaccatcctggaagctggtgacgaactgctg
gttcgtgttctgggtcgtcgtatcggtgcttggctgcgtggtctgctgaccgaactgggtgttcaggttgaactgggtaccggtgttgttgg
tttctctggtgaaggtcagctggaacaggttatggcttctgacggtcgttctttcgttgctgactctgctctgatctgcgttggtgctgaacc
ggctgaccagctggctcgtcaggctggtctggcttgcgaccgtggtgttatcgttgaccactgcggtgctaccctggctaaaggtgttttc
gctgttggtgacgttgcttcttggccgctgcgtgctggtggtcgtcgttctctggaaacctacatgaacgctcagcgtcaggctgctgctg
ttgctgctgctatcctgggtaaaaacgtttctgcgccgcagctgccggtgagctggaccgaaatcgctggtcaccgtatgcagatggct
ggtgacatcgaaggtccgggtgacttcgtttctcgtggtatgccgggttctggtgctgctctgctgttccgtctgcaagaacgtcgtatcc
aggctgttgttgctgttgacgctccgcgtgacttcgctctggctacccgtctggttgaagctcgtgctgctatcgaaccggctcgtctggct
gacctgtctaactctatgcgtgacttcgttcgtgctaacgaaggtgacctgacctaa

## Claims

1. A polynucleotide, comprising coding sequences of toluene dioxygenase TodC1, TodC2, TodB and TodA, wherein the polynucleotide comprises, from 5'-end to 3'-end:
(1) a first nucleotide sequence comprising the coding sequence of TodC1, wherein the TodC1 has an amino acid sequence of SEQ ID NO: 4;
(2) a second nucleotide sequence comprising the coding sequence of TodC2, wherein the TodC2 has an amino acid sequence of SEQ ID NO: 5;
(3) a third nucleotide sequence comprising the coding sequence of TodB, wherein the TodB has an amino acid sequence of SEQ ID NO: 6; and
(4) a fourth nucleotide sequence comprising the coding sequence of TodA, wherein the TodA has an amino acid sequence of SEQ ID NO: 7;
wherein the first, second, third and fourth nucleotide sequences each independently use *E*. *coli* preferred codons, and the stop codon TAA for TodB and the start codon ATG for TodA form a nested sequence TAATG.

2. The polynucleotide according to claim 1, wherein
the first nucleotide sequence comprises nucleotides at positions 1-1350 of SEQ ID NO: 1; and/or
the second nucleotide sequence comprises nucleotides at positions 1464-2024 of SEQ ID NO: 1; and/or
the third nucleotide sequence comprises nucleotides at positions 2036-2356 of SEQ ID NO: 1; and/or
the fourth nucleotide sequence comprises nucleotides at positions 2359-3588 of SEQ ID NO: 1.

3. The polynucleotide according to claim 1 or 2, wherein
the polynucleotide further comprises a first spacer sequence located between the first nucleotide sequence and the second nucleotide sequence, and/or a second spacer sequence located between the second nucleotide sequence and the third nucleotide sequence,
wherein the first spacer sequence comprises, from 5'-end to 3'-end, a T7 promoter, a Lac operator and a prokaryotic ribosome binding site (rbs), and/or the second spacer sequence comprises an rbs sequence rich in AG;
preferably, the first spacer sequence comprises a nucleotide sequence of SEQ ID NO: 3, and/or
the second spacer sequence comprises GTGATGTC (nucleotides at positions 2028-2035 of SEQ ID NO: 1) or GAAGGAGATATACC (nucleotides at positions 2028-2041 of SEQ ID NO: 2).

4. The polynucleotide according to claim 3, comprising a nucleotide sequence of SEQ ID NO: 1 or SEQ ID NO: 2.

5. An expression cassette or vector, comprising the polynucleotide according to any one of claims 1-4.

6. The expression cassette or vector according to claim 5, comprising, from 5'-end to 3'-end:
(1) a second T7 promoter (preferably having a nucleotide sequence of SEQ ID NO: 8);
(2) the polynucleotide according to any one of claims 1-4; and
(3) an optional T7 transcription terminator (preferably having a nucleotide sequence of SEQ ID NO: 11);
preferably, the expression cassette or vector further comprises a second prokaryotic ribosome binding site located between the second T7 promoter and the first nucleotide sequence (preferably, the second prokaryotic ribosome binding site has a nucleotide sequence of SEQ ID NO: 9);
more preferably, the expression cassette or vector further comprises a second Lac operator located between the second T7 promoter and the second prokaryotic ribosome binding site (preferably, the second Lac operator has a nucleotide sequence of SEQ ID NO: 10).

7. The vector according to claim 5, which is derived from a vector selected from the group consisting of pET-3a-d, pET-9a-d(+), pET-11a-d, pET-14b, pET-21a-d(+), pET-23a-d(+), pET-24a-d(+), pET-30a-c(+), pET-28a-c(+), pET31b(+), pET32a-c(+), p33b, pET-41a-c(+) and pET-42a-c(+).

8. A host cell, comprising the polynucleotide according to any one of claims 1-4, or the expression cassette or vector according to any one of claims 5-7;
preferably, the host cell is *E. coli* expressing a T7 RNA polymerase;
more preferably, the host cell is selected from the group consisting of *E. coli* BL21(DE3), BL21-Gold (DE3), BL21-Gold (DE3)pLysS, Rosetta (DE3), Origami^{™} 2 (DE3), OrigamiB (DE3), Origami^{™} B (DE3)pLysS, Rosetta-gami B (DE3) and Lemo21 (DE3).

9. A process for preparing a toluene dioxygenase, comprising
culturing the host cell according to claim 8 under conditions suitable for the expression of the expression cassette or vector.

10. The process according to claim 9, further comprising lysing the host cell expressing the toluene dioxygenase to give a lysate comprising the toluene dioxygenase;
preferably the process further comprises purifying the toluene dioxygenase.

11. Use of the polynucleotide according to any one of claims 1-4, the expression cassette or vector according to any one of claims 5-7, or the host cell according to claim 8 for preparing a compound of formula (2) 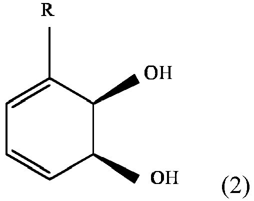 wherein
R is selected from the group consisting of halogen, -C₁₋₆ alkyl, -C₂₋₆ alkenyl, -C₁₋₆ alkyl -CN, -C₁₋₆ alkyl-O-C(=O)-C₁₋₆ alkyl, -C₃₋₈ cycloalkyl, -C₃₋₈ cycloalkenyl, and -C₆₋₁₀ aryl,
wherein the C₁₋₆ alkyl, -C₂₋₆ alkenyl, -C₁₋₆ alkyl-CN, -C₁₋₆ alkyl-O-C(=O)-C₁₋₆ alkyl, -C₃₋₈ cycloalkyl, -C₃₋₈ cycloalkenyl, and -C₆₋₁₀ aryl are optionally substituted with one or more halogen;
preferably, R is selected from the group consisting of halogen, methyl, -CH=CH₂, -CH=CHBr, -CH₂CH₂Br, -CH₂CH₂CN, -CH₂CH₂N₃, -CH₂CH₂-O-C(=O)-CH₃, -CH₂-O-C(=O)-CH₃, cyclohexyl, cyclohexenyl, and phenyl.

12. A process for preparing a compound of formula (2), 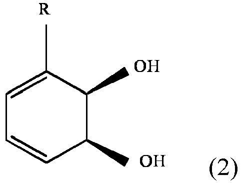 the process comprising,
(1) culturing the host cell according to claim 8 under conditions suitable for the expression of the expression cassette or vector;
(2) adding a compound of formula (1) as substrate to the culture medium containing the host cell of step (1) for reaction to give a fermentation broth containing the compound of formula (2), 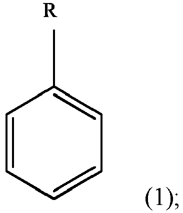 and
(3) recovering the compound of formula (2) from the fermentation broth of step (2); wherein R is as defined in claim 11.

13. The process according to claim 12, wherein
R is methyl, the compound of formula (1) is toluene and the compound of formula (2) is cis-(1S,2R)-3-methyl-3,5-cyclohexadiene-1,2-diol; or
R is I, the compound of formula (1) is iodobenzene and the compound of formula (2) is cis-(1S,2R)-3-Iodo-3,5-cyclohexadiene-1,2-diol; or
R is -CH₂-O-C(=O)-CH₃, the compound of formula (1) is benzyl acetate and the compound of formula (2) is cis-(1S,2R)-3-acetoxymethyl-3,5-cyclohexadiene-1,2-diol.

14. Use of the polynucleotide according to any one of claims 1-4, the expression cassette or vector according to any one of claims 5-7, or the host cell according to claim 8 for preparing Tetrodotoxin.
